# EUROPEAN PATENT APPLICATION

(11) **EP 1 065 047 A1**
(43) Date of publication of application: **03.01.2001**
(21) Application number: 00305454.1
(22) Date of filing: 29.06.2000
(51) Int. Cl.: B32B 7/14, A61F 13/15, B32B 31/00

(54) **Disposable laminated panel**

(30) Priority: 29.06.1999 JP 18342199
(71) Applicant: UNI-CHARM CORPORATION, Kawanoe-shi Ehime-ken (JP)
(72) Inventor: Miyazaki, Yuka, C/o Technical Center, Mitoyo-gun, Kagawa-ken 769-1602 (JP); Yoshioka, Toshiyasu, C/o Technical Center, Mitoyo-gun, Kagawa-ken 769-1602 (JP)
(74) Representative: Parry, Christopher Stephen

(57) **Abstract**

A disposable laminated panel 1 including at least two panel members placed upon and joined to each other by means of adhesive B1, B2 applied on at least one of their surfaces facing each other, wherein the adhesive B1, B2 forms a plurality of independent joining lines undulating in one direction so that these joining lines might neither intersect one another nor intersect themselves.

## Description

This invention relates to a disposable laminated panel comprising at least two sheet members laminated one upon another and more particularly relates to such a laminated panel suitable for use in articles such as disposable diaper covers, disposable diapers, sanitary napkins and absorbent incontinent pads.

Japanese Patent Application Disclosure No. 1996-196559 describes a disposable diaper in which a liquid-absorbent core comprising a liquid-holding layer, a fibrous assembly layer underlying the liquid-holding layer and a liquid-pervious sheet such as tissue paper covering these two layers. This known disposable diaper is characterized in that these three components are joined together by means of hot melt adhesive intermittently applied on substantially entire surfaces over which the three components are placed one upon another. The gazette describes various patterns in which the hot melt adhesive may be applied on the surfaces as follow: a plurality of dotted straight lines extending parallel one to another transversely of the diaper; a plurality of straight lines extending in parallel one to another longitudinally of the diaper; a plurality of straight lines intersecting one another to form cross stripes; and a plurality of spirals, each intersecting itself at regular pitches, extending in one direction of the diaper.

Adhesive applied in the pattern of dotted lines extending transversely of the diaper as well as in the pattern of cross stripes extending longitudinally of the diaper is effective to prevent the core and the sheet from being separated from each other in the direction orthogonal to the direction in which these dotted lines or straight lines extend. However, the core and the sheet may be easily separated from each other so far as the direction in which these lines of adhesive extend is concerned because the adhesive can resist separation between the core and the sheet not along continuous lines but at intermittent points.

Adhesive applied in the pattern of cross stripes as well as in the pattern of spirals each intersecting itself at regular pitches may be accompanied with serious problem. Specifically, the adhesive is twice applied at respective intersections and a total amount of adhesive inevitably increases. Such excessive amount of adhesive may exude over the outer surface of the sheet or spread over the inner surface of the sheet. The amount of adhesive exuding over the outer surface of the sheet may stick to surfaces of pressure rolls used to compress the core to a desired thickness and consequently the sheet also may stick the pressure rolls. The amount of adhesive spreading over the inner surface may cover the core with the adhesive and deteriorate the liquid-absorbent ability of the core.

It is an object of this invention to provide a laminated panel members for a disposable garment comprising at least two panel members together by application of adhesive in patterns adapted to prevent the members from being easily separated from each other regardless of the adhesive pattern's direction, on one hand, and to prevent the adhesive from exuding over the outer surfaces of the members and/or spreading over the inner surfaces of the members, on the other hand.

According to this invention, there is provided a disposable laminated panel member comprising at least two panel members placed upon and joined to each other by means of adhesive applied on at least one of surfaces thereof facing each other, and the adhesive forming a plurality of independent joining lines undulating in one direction so that the joining lines neither intersect one another nor intersect themselves.

According to one preferred embodiment of this invention, the panel members comprise a liquid-pervious topsheet and a liquid-impervious backsheet, wherein a liquid-absorbent core is disposed between the topsheet and the backsheets and wherein the topsheet, the backsheets and the core are joined together by the adhesive applied at least one of each pair of surfaces thereof facing each other.

According to another preferred embodiment of this invention, the panel members comprise moisture-pervious sheets disposed between the topsheet and the backsheet to cover the core and wherein the core and the moisture-pervious sheets are joined together by the adhesive applied on surfaces of respective the moisture-pervious sheets facing the core.

According to still another preferred embodiment of this invention, at least one of the topsheet and the backsheet extends laterally outward beyond transversely opposite side edges of the core to form a pair of longitudinally extending side flaps, wherein the topsheet and the backsheet longitudinally extending outward beyond longitudinally opposite ends of the core to form a pair of transversely extending end flaps, wherein the side flaps are provided with a pair of longitudinally stretchable elastic members, respectively, so as to be associated with a pair of leg-openings, wherein at least one of the end flaps is provided with a transversely stretchable elastic member so as to be associated with a waist-opening and wherein the elastic members associated with the leg-openings and said elastic member associated with the waist-opening are secured under tension to the inner surface of at least one of the topsheet and the backsheet by the adhesive.

According to further another preferred embodiment of this invention, the adhesive is hot melt adhesive.
Fig. 1 is an exploded perspective view showing a disposable diaper as an embodiment of this invention;
Fig. 2 is a partially cutaway perspective view showing the diaper in its assembled state; and
Fig. 3 is a sectional view taken along line A - A in Fig. 2.

Details of a laminated panel used in a disposable garment according to this invention will be more fully understood from the description of a disposable diaper as one embodiment of this invention given hereunder with reference to the accompanying drawings.

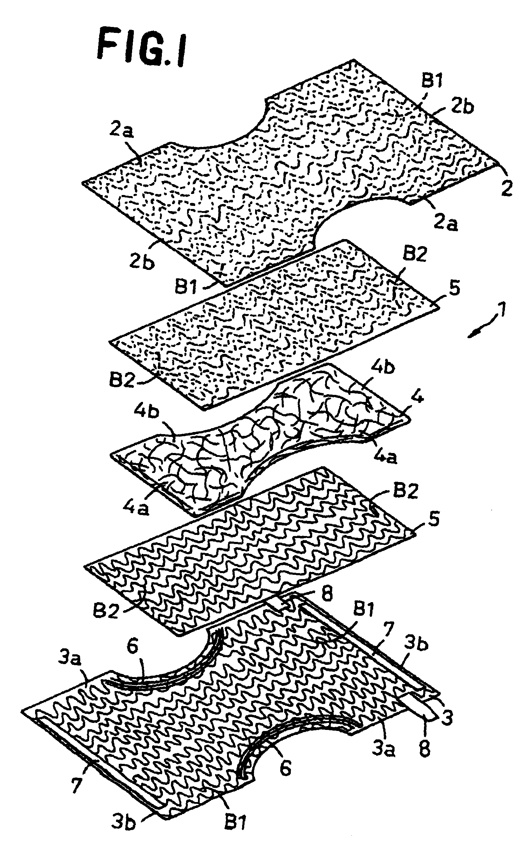

Fig. 1 is an exploded perspective view showing a disposable diaper. The diaper is in the form of a laminated panel 1 comprising an hourglass-shaped liquid-perviouS topsheet 2, an hourglass-shaped liquid-impervious backsheet 3, a pair of tissue paper sheets 5, 5 (liquid-pervious sheets) and an hourglass-shaped liquid-absorbent core 4 disposed between the pair of tissue paper sheets 5, 5.

As will be apparent from Fig. 1, the panel 1 comprises the backsheet 3, one of the tissue paper sheets 5, the core 4, the other tissue paper sheet 5 and the topsheet 2 layered in this order starting from the backsheet 3 as the lowermost layer. The topsheet 2 and the backsheet 3 are substantially the same in shape as well as in size and respectively contoured by transversely opposite side edges 2a, 2a; 3a, 3a extending longitudinally and partially curved transversely inward in longitudinally middle zones of these sheets 2, 3 and longitudinally opposite ends 2b, 2b; 3b, 3b extending transversely of these sheets 2, 3. Each of the tissue paper sheets 5 is of rectangular shape having its area smaller than the topsheet 2 and the backsheet 3 and larger than the core 4.

The backsheet 3 is provided on its inner surface with a pair of elastically stretchable members 6, 6 extending along the transversely opposite side edges 3a, 3a, respectively, and secured under tension to the backsheet 3 so that these members 6, 6 may be associated with respective leg-openings. The backsheet 3 is further provided on its inner surface with a pair of elastically stretchable film-like members 7, 7 extending along the longitudinally opposite ends 3b, 3b and secured under tension to the backsheet 3 so that these film-like members 7, 7 may be associated with a waist-opening. Between the elastic members 6, 6 for the leg-openings and the elastic members 7, 7 for the waist-opening, the backsheet 3 is provided on its respective side edges 3a, 3a with a pair of tape fasteners 8, 8. These tape fasteners have their proximal ends fixed to the inner surface of the backsheet 3 so that the respective tape fasteners extend transversely outward from the side edges 3a, 3a.

The topsheet 2 and the backsheet 3 are applied on their surfaces opposed to each other with hot melt adhesive B1 by means of which these topsheet 2 and the backsheet 3 are joined not only to each other but also to the pair of tissue paper sheets 5, 5, respectively. The pair of tissue paper sheets 5, 5 are applied on their inner surfaces with hot melt adhesive B2 by means of which the pair of tissue paper sheets 5, 5 are joined to the core 4. The adhesive B1, B2 is applied on the topsheet 2 and the backsheet 3 and the pair of tissue paper sheets 5, 5 substantially with evenness.

The adhesive B1, B2 form a plurality of independent joined lines undulating longitudinally of the panel 1 and extending so that these bending lines might neither intersect one another nor intersect themselves.

Referring to Fig. 1, the core 4 may be entirely covered with the pair of tissue paper sheets 5, 5 from above and below to join the core 4 to the pair of tissue paper sheets 5, 5 by means of the adhesive B2 applied on the inner surfaces of the respective tissue paper sheets 5, 5. Now the topsheet 2 and the backsheet 3 may be placed upon the respective outer surfaces of the tissue paper sheets 5, 5 to join the pair of tissue paper sheets 5, 5 to the topsheet 2 and the backsheet 3 respectively, by means of the adhesive B1 applied on the respective inner surfaces of the topsheet 2 and the backsheet 3 and, at the same time, to join the topsheet 2 and the backsheet 3 to each other along their transversely opposite side edges 2a, 2a; 3a, 3a and their longitudinally opposite ends 2b, 2b; 3b, 3b. The elastic members 6 for the leg-openings and the elastic members 7 for the waist-opening are joined to the respective inner surfaces of the topsheet 2 and the backsheet 3 as these topsheet 2 and the backsheet 3 are joined to each other.

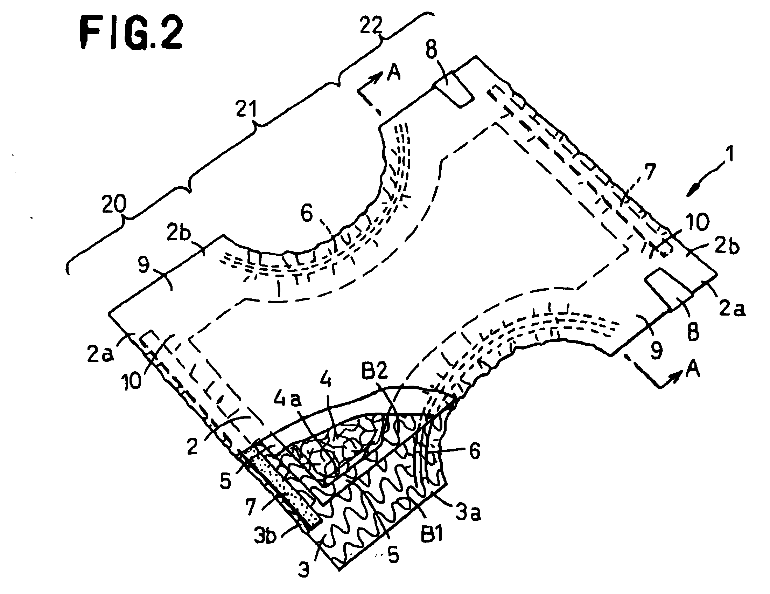

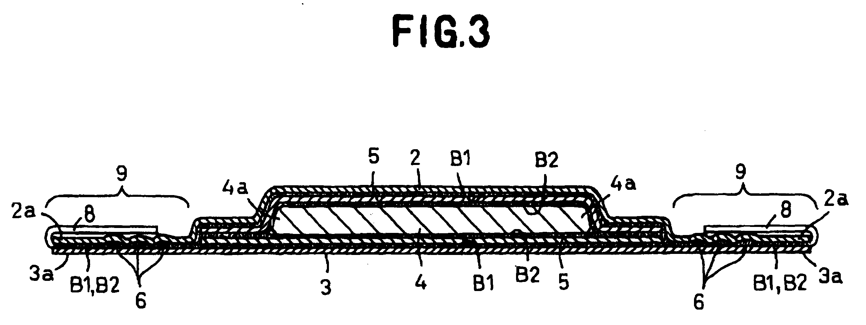

Fig. 2 is a partially cutaway perspective view showing the diaper in its assembled state and Fig. 3 is a sectional view taken along line A - A in Fig. 2. The panel 1 is longitudinally composed of a front waist region 20, a rear waist region 22 and a crotch region 21 extending between these two waist regions 20, 22. The panel 1 has a pair of transversely opposite side flaps 9 extending longitudinally and curved inward transversely of the panel 1 in the crotch region 21 and a pair of longitudinally opposite end flaps 10 extending transversely of the panel 1.

The side flaps 9, 9 of the panel 1 are formed by the side edges 2a, 2a; 3a, 3a of the topsheet 2 and the backsheet 3 extending transversely outward beyond transversely opposite side edges 4a, 4a of the core 4. Along these side flaps 9, 9, the topsheet 2 and the backsheet 3 are joined to each other by means of the adhesive B1 applied on the respective inner surfaces of these sheets 2, 3 and the elastic members 6, 6 for the leg-opening disposed between these sheets 2, 3 are joined to the respective inner surfaces of these sheets 2, 3.

The end flaps 10, 10 of the panel 1 are formed by the ends 2b, 2b; 3b, 3b of the topsheet 2 and the backsheet 3 extending longitudinally outward from longitudinally opposite ends 4b, 4b of the core 4. Along these end flaps 10, 10, the topsheet 2 and the backsheet 3 are joined to each other by means of the adhesive B1 applied on the respective inner surfaces of these sheets, 2, 3 and the elastic members 7, 7 for the waist-opening disposed between these sheets 2, 3 are secured to the respective inner surfaces of these sheets 2, 3.

In the rear waist region 22 of the panel 1, the tape fasteners 8, 8 are folded back to extend inward transversely of the panel 1 and have their free ends separably fixed to the outer surface of the topsheet 2 by means of adhesive (not shown) applied on the respective free ends of the tape fasteners 8, 8. Gathers are formed along the side flaps 9, 9 and the end flaps 10, 10 of the panel 1 as the elastic members 6, 6 for the leg-openings and the elastic members 7, 7 for the waist-opening are relaxed to contract.

In the sectional view of Fig. 3, the adhesive B1, B2 applied on the respective inner surfaces of the topsheet 2 and the backsheet 3 and the pair of tissue paper sheets 5, 5 appear not as being intermittent but as being substantially continuous transversely of the panel 1.

Though not illustrated, it is possible without departing from the scope of this invention to form the side flaps 9, 9 of the panel 1 by the side edges 3a, 3a of the backsheet 3 and a pair of liquid-resistant sheets provided along the side edges 3a, 3a of the backsheet 3 so as to extend longitudinally of the panel 1. In this case, the liquid-resistant sheets are applied on their inner surfaces with the adhesive B1 in the same pattern as the adhesive applied on the topsheet 2 and the backsheet 3. By means of such adhesive B1, the liquid-resistant sheets are joined to the outer surface of the topsheet 2 along its side edges 2a, 2a terminating immediately outside of the side edges 4a, 4a of the core 4 and to the inner surface of the backsheet 3 along its side edges 3a, 3a extending outward beyond the side edges 2a, 2a of the topsheet 2 transversely of the panel 1. The elastic members 6, 6 for the leg-openings are disposed between the side edges 3a, 3a of the backsheet 3 and the liquid-resistant sheets, respectively, and secured to the inner surfaces thereof. In the case of the panel 1 having the side flaps 9, 9 formed by the backsheet 3 and the liquid-resistant sheets, even when an amount of body fluids exudes into the side edges 2a, 2a of the topsheet 2, leakage of body fluids from the side flaps 9, 9 can be effectively prevented because the amount of body fluids can not further exude into the backsheet 3 and the liquid-resistant sheets.

The topsheet 2 may be formed by a hydrophobic nonwoven fabric treated by suitable agent making it hydrophilic or a hydrophilic nonwoven fabric made of fiber compounded with the agent making it hydrophilic. It is also possible to use a thermoplastic synthetic resin film having a plurality of pores in the place of such nonwoven fabric. The backsheet 3 and the liquid-resistant sheets are formed by a synthetic resin film or a laminate of a synthetic resin film and a hydrophobic nonwoven fabric, preferably by a breathable but liquid-impervious sheet. The nonwoven fabric used for such purpose may be selected from a group including an air-through nonwoven fabric, a point bond nonwoven fabric, a spun bond nonwoven fabric, a spun lace nonwoven fabric and a melt blown nonwoven fabric.

The core 4 is formed by a mixture of fluff pulp and highly absorptive polymer particles or such mixture added with fiber functioning to maintain a desired shape, which is then compressed to a desired thickness. The elastic members 6, 7 may be formed by material such as synthetic rubber or natural rubber, or strips of nonwoven fabric to which the material has been secured under tension.

The hot melt adhesive B1, B2 may be hot melt adhesive of block polymer rubber type or hot melt adhesive of olefine type. The adhesive B1, instead of being applied on both the topsheet 2 and the backsheet 3, may be also applied on one of the topsheet 2 and the backsheet 3. It is also possible to apply the topsheet 2 and the backsheet 3 and the pair of tissue paper sheets 5, 5 in a pattern of dotted lines.

To join the pair of tissue paper sheets 5, 5 to the core 4 disposed between them, an amount of the adhesive B2 to be applied on the pair of tissue paper sheets 5, 5 is preferably in a range of 1 g/m² - 5 g/m². To join the topsheet 2 and the backsheet 3 to each other, an amount of the adhesive B1 to be applied on these topsheet 2 and the backsheet 3 is preferably in a range of 3 g/m² - 8 g/m². To join the elastic members 6, 7 to the topsheet 2 and the backsheet 3, an amount of the adhesive B1 to be applied on the topsheet 2 and the backsheet 3 is preferably in a range of 8 g/m² - 15 g/m². The amount of adhesive less than these ranges may result in an adhesive force insufficient to hold the component members such as the sheets 2, 3, the elastic members 6, 7, the core 4 and the pair of tissue paper sheets 5, 5 together in the form of the laminated panel 1. Depending on the particular basis weights of the nonwoven fabric and the tissue paper sheets 5, 5 as well as the particular fineness of the fiber constituting the nonwoven fabric, the amount of the adhesive larger than the ranges may cause also serious troubles. Specifically, an excessive amount of the adhesive may exude over the outer surfaces and/or spread over the inner surfaces of the sheets 2, 3 and the tissue paper sheets 5, 5.

Application of this invention is not limited to the disposable diaper but applicable also to the other various products such as diaper covers, sanitary napkin, absorbent incontinent pads, a composite sheet for wipe-out, and a composite sheet comprising a non-stretchable sheet and a stretchable sheet secured under tension to the non-stretchable sheet.

As will be appreciated from the foregoing description, the component members of the disposable laminated panel according to this invention such as the topsheet and the backsheet and tissue paper sheets are applied with the adhesive in the unique pattern comprising a plurality of independent lines extending in one direction so as to form independent undulations. Such pattern of adhesive lines ensures an adhesive force substantially uniform along the direction of the adhesive lines as well as the direction transversely of the adhesive lines and sufficient to hold the topsheet and the backsheet, the elastic members, the pair of tissue paper sheets and the core together in the form of a laminated panel.

Furthermore, the adhesive is applied as evenly as possible on the topsheet and the backsheet and the tissue paper sheets in the pattern such that the adhesive lines might neither intersect one another nor intersect themselves. Therefore, there is no apprehension that the amount of adhesive might locally increase and an excessive amount of the applied adhesive might exude over the outer surfaces and/or spread over the inner surfaces of the topsheet and the backsheet and the tissue paper sheets.

## Claims

1. A disposable laminated panel comprising at least two panel members placed upon and joined to each other by means of adhesive applied on at least one of surfaces thereof facing each other, and said adhesive forming a plurality of independent joining lines undulating in one direction so that said joining lines neither intersect one another nor intersect themselves.

2. A disposable laminated panel according to Claim 1, wherein said panel members comprise a liquid-pervious topsheet and a liquid-impervious backsheet, wherein a liquid-absorbent core is disposed between said topsheet and said backsheets and wherein said topsheet, said backsheets and said core are joined together by said adhesive applied at least one of each pair of surfaces thereof facing each other.

3. A disposable laminated panel according to Claim 2, wherein said panel members comprise moisture-pervious sheets disposed between said topsheet and said backsheet to cover said core and wherein said core and said moisture-pervious sheets are joined together by said adhesive applied on surfaces of respective said moisture-pervious sheets facing said core.

4. A disposable laminated panel according to Claim 2, wherein at least one of said topsheet and said backsheet extends laterally outward beyond transversely opposite side edges of said core to form a pair of longitudinally extending side flaps, wherein said topsheet and said backsheet longitudinally extending outward beyond longitudinally opposite ends of said core to form a pair of transversely extending end flaps, wherein said side flaps are provided with a pair of longitudinally stretchable elastic members, respectively, so as to be associated with a pair of leg-openings, wherein at least one of said end flaps is provided with a transversely stretchable elastic member so as to be associated with a waist-opening and wherein said elastic members associated with the leg-openings and said elastic member associated with the waist-opening are secured under tension to the inner surface of at least one of said topsheet and said backsheet by said adhesive.

5. A disposable laminated panel according to Claim 1, wherein said adhesive is hot melt adhesive.

6. A laminated panel according to Claim 1, wherein said panel members comprise a nonwoven fabric.
